# EUROPEAN PATENT APPLICATION

(11) **EP 2 327 395 A1**
(43) Date of publication of application: **01.06.2011**
(21) Application number: 09176713.7
(22) Date of filing: 23.11.2009
(51) Int. Cl.: A61K 9/08, A61K 49/00, C07C 227/38, C07C 229/22

(54) **Process for the preparation of gadobenate dimeglumine complex in a solid form**

(71) Applicant: Bracco Imaging S.p.A, 20134 Milano (IT)
(72) Inventor: Fretta, Roberta, 1-10010 Ivrea (IT); Ferrigato, Aurelia, 1-10010 Ivrea (IT); Lattuada, Luciano, 1-10010 Ivrea (IT); Morosini, Pier, Francesco, 26818 Lodi (IT); Ceragioli, Silvia, 1-10010 Ivrea (IT); Uggeri, Fulvio, 1-10010 Ivrea (IT); Anelli, Pier, Lucio, 1-10010 Ivrea (IT)
(74) Representative: Macchetta, Francesco

(57) **Abstract**

The present invention discloses a process for the preparation of gadobenate dimeglumine complex in a solid form. In particular, said solid form is conveniently obtained by spray-drying a corresponding liquid suspension at a given temperature and concentration. The present invention is particularly advantageous for the industrial scale as the solid form may be obtained by employing water as a solvent, which is a non-toxic solvent, easy to handle and basically not requiring troublesome health or safety precautions.

## Description

The present invention generally relates to a process for the preparation of a solid form of a polyamino polycarboxylic gadolinium complex by a spray-drying procedure. In more details, the present process enables for the collection of a solid form of the gadobenate dimeglumine complex, which may be employed, for example, as a contrast agent in the diagnostic imaging field.

### Background

Contrast media are substances used to enhance the contrast of structures or fluid within the body in the medical imaging field. Among the imaging techniques currently employed, the magnetic resonance imaging (MRI) is one of the most relevant, due to its efficacy and safety and, in this prospect, several contrast media have been developed during the last decades. Said MRI contrast media are, basically, constituted by a paramagnetic metal (generally gadolinium) which is complexed with a poliammino carboxylic chelate, either cyclic or acyclic. Examples of said paramagnetic complexes are Gd-DTPA, Gd(HP-D03A) and Gd-BOPTA. In particular, the physiologically compatible salt of this latter (*i.e.* the dimeglumine salt see The Merck Index, XII Ed., 2001, Nr 4344), referred also as gadobenate dimeglumine complex, of Formula I below, constitutes the active ingredient of one of the most commonly used MRI contrast agent, commercially known as MultiHance® by Bracco Imaging SpA.

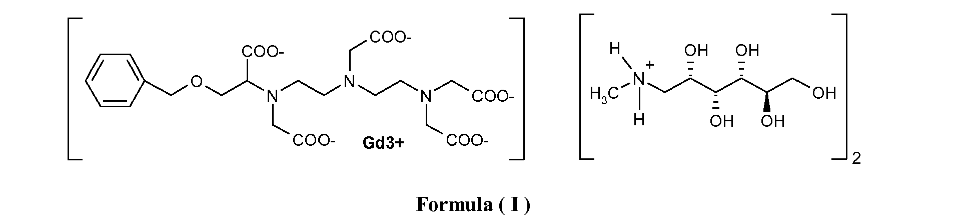

By now, MultiHance® is actually obtained in a proper liquid form, ready for the administration, as disclosed in EP0230893.

It has to be noted that in the preparation of pharmaceutical compounds, and specifically in the manufacturing of compounds intended to be used as contrast agents, the degree of purity as well as several other quality standards are very restricted, according to the competent authorities requirements.

In the imaging field, accordingly, the contrast agent has to be prepared in a pure, stable and convenient physical form and, in the most of the cases, this represents a crucial point and a challenge that any manufacturer has to face. A suitable physical form should be the one that allows, first of all, a reliable and practical recovering of the final compound and, secondly, it should guarantee an easy storage of the product, substantially retaining the physical-chemical properties of the solid along the time.

In this respect, and whenever possible, the solid form is generally preferred and when the product is obtained, for example at the end of a synthetic process, in a liquid or oily form or in a solution or suspension thereof, several isolation and purification techniques are commonly employed in order to convert such a product into a corresponding solid form (see for instance, Huang et al., Advanced Drug Delivery Reviews; 2004; 56; 321-334).

Among the procedures known to the skilled in the art, the selective precipitation from a proper solution, the evaporation of the solvent, the lyophilisation and the crystallization from a suitable organic solvent, or from a mixture of solvents, are some examples of methodologies widely used to this extent (see for example: TUMJ; 2001, 59(3), 53-59 and Palermo et al. Chemical Reviews; 1968; Vol. 60; 65-93).

The cited procedures, taken alone or even in any combination thereof, are currently employed, from the bench to the industrial scale, in those cases where a final solid form of a compound is desirable or required, for example for reasons of workability or for the preparation of a solid drug dosage form. Typically, a filtration and a final drying step, usually under reduced pressure, are carried out in order to collect the product as a dried solid (see for a general reference, Takashi et al. Journal of the Society of Powdered Technology, 2006; Vol.43; No12; 882-889).

Spray-drying technique represents an alternative method to the above, where a solid compound is collected starting from a proper solution (habitually, an aqueous solution) or a suspension of the same, by a spray-dryer device. This technique, however, suffers from some drawbacks, in particular when applied to molecules with specific chemical features, such as for instance, organic complexes or the like. In more detail, in fact, some structural and/or chemical physical changes may be observed, including, among others, polymorphic changes, solvate formation and further, the obtainment of a product in an undesired or glassy form (for a general reference, see for example, Corrigan et al., Thermochimica Acta 248; 1995; 245-258).

We have now found that when a proper liquid composition of the gadobenate dimeglumine complex is subjected to a spray-dry procedure, the solid form thus obtained may be conveniently collected in high and reproducible yields.

### Summary of the invention

It is therefore a first aspect of the present invention a process for the preparation of a solid form of the gadobenate dimeglumine compound, which process comprises spray-drying a liquid composition of said compound. Preferably, the liquid composition is an aqueous composition selected from a solution or a suspension.

Noteworthy, the solid form obtained according to the invention substantially retains the specifications of the starting liquid composition, even when the latter is intended for the administration and therefore in conformity with the limits and the specifications as required.

Analogously and according to another aspect, the present invention relates to a process for the preparation of a solid form of BOPTA, characterised by spray-drying a liquid composition of such compound.

### Detailed description of the invention

The process of the present invention advantageously enables for the preparation of a suitable solid form of the gadobenate dimeglumine, differently from the product obtainable by using other commonly employed methodologies that actually lead to a solid form with unfavourable handling and workability (gummy, sticky or glassy solids). Said solid form, in particular, presents a good flowability, a good stability, a low hygroscopicity and a low electrostaticity, along with a controlled particle size distribution and a high quality, as no impurity formation or thermal degradation is detected throughout the process.

The present invention is particularly advantageous for the industrial scale as the solid form may be obtained by employing water as a solvent, which is a non-toxic substance, easy to handle and basically not requiring troublesome health or safety precautions. Unless otherwise provided, with the term "solid form" we mean not solved or suspended in any media.

Practically, in the spray-drying device, a liquid composition containing the active ingredient is subjected to an atomization process, dried inside the spray-drying chamber by using a drying gas at a controlled temperature, and finally the thus formed particles are collected throughout a cyclone.

The term "atomization process" is intended to indicate the formation of micro-particles, usually in the form of micro-drops or the like.

The term "liquid composition" refers to a solution, or a suspension, of the selected compound in any appropriate solvent system.

Typical examples of said solvent systems are, *inter alia,* aqueous systems such as purified water (*e.g*. demineralised, distilled or deionised water and the like), or a mixture of water and a water-miscible solvent. Examples of water-miscible solvents are, for instance, polar solvents, including, but not limited to, lower (e.g. C₁-C₄) alcohols, acetone, and the like.

If not stated otherwise, the gadobenate dimeglumine may be present in the liquid composition in any suitable amount, for example so that the nozzle obstruction is prevented. In case of solutions, examples of suitable concentration solution may be for instance up to 0.6 M, preferably from 0.2 to 0.6 M, whereas the most preferred one is 0.5 M (with M meaning the molarity of the solution).

In more detail, in the present process the liquid composition may be fed to the apparatus at room temperature (*i.e*. about 20-25°C, or even at higher temperatures), and submitted to an atomization process in the spray-dryer chamber by using a known atomisation device (located for example at the top of said chamber). Examples of suitable atomization devices comprise, among others, a pressure, a rotary, or a two fluid nozzle. Particularly preferred are either the two fluid nozzle or the pressure nozzle.

As regards the liquid composition fed rate, it may be properly chosen as the case may be, and preferred values are, for example, from about 5 to 13 g/min, more preferably from about 8 to 10 g/min. In case of a possible employment of the present process on industrial scale, for example in a pilot plant, the feed rate may be comprised between 2500 and 7500 g/h, preferably around 3000 g/h.

The thus atomised liquid composition is then dried in the device by using a co-current flow of a drying gas commonly known in the art for similar procedures, such as, for instance, air or nitrogen. According to the invention the inlet temperatures of the gas (herein referred as Tinlet) lies in the range of about 140°C to 280°C, preferably the inlet temperature lies between 160 and 200°C.

Accordingly, the outlet temperature (herein indicated as Toutlet) of the gas lies between about 70°C to about 120°C.

By passing through a cyclone, the gadobenate dimeglumine is recovered in a solid form, typically in an amorphous solid form, in high yields of conversion (up to 98%) and with a content of residual water comprised between 0.7 and 5.5% (calculated by Karl Fisher titration and herein indicated as KF) as detailed in the experimental part herein below.

Whenever required, said solid may be appropriately stored using precautions known in the art (*e.g.* protective packaging such as moisture proof bags), satisfactorily avoiding the formation of side products or alterations of the physical-chemical properties of the solid. The latter, may be readily used in the preparation of pharmaceutical compositions, for example, in the preparation of an injectable formulation to be used as MRI contrast agent.

In a particular embodiment of the invention, the solid gadobenate dimeglumine may advantageously be packaged in a two component diagnostic and/or therapeutic kit, preferably for administration by injection. The kit may comprise a first container, containing the solid gadobenate dimeglumine, and a second container, containing a physiologically acceptable aqueous carrier. Examples of suitable carries are water, typically sterile, pyrogens free water (to prevent as much as possible contamination in the solid product, and also generally indicated as water for injection), aqueous solution such as saline (which may advantageously be balanced so that the final product for injection is not hypotonic), or aqueous solutions of one or more tonicity adjusting substances such as salts or sugars, sugar alcohol, glycols or other non-ionic polyol materials (e.g. glucose, sucrose, glycerol, glycols and the like). Said two component kits can include two separate containers or a dual-chamber container. In the former case the container is preferably a conventional septum-sealed vial, wherein the vial containing the solid residue is sealed with a septum through which the liquid carrier may be injected using an optionally prefilled syringe. In such a case, the syringe used as the container of the second component is also used then for injecting the contrast agent. In the latter case, the dual chamber container is preferably a dual chamber syringe and once the solid has been reconstituted and then suitably mixed or gently shaken, the container can be used directly for injecting the contrast agent.

The process of the present invention may be carried out by using a spray-drying equipment or plant, selected from those commercially available, such as, for instance, LAB PLANT SD 04 spry dryer or, alternatively in case of industrial scale amounts, a MOBILE MINOR^{™} pilot plant.

As regards to the starting material, a liquid composition of the gadobenate dimeglumine might be easily prepared by reacting the polyamino carboxylic derivative 4-carboxy-5,8,11-tris(carboxymethyl1)-1-phenyl-2-oxa-5,8,11-triazatridecan-13-oic acid (BOPTA) as the ligand, with Gd₂O₃ and the N-methyl-glucamine (meglumine), as disclosed, for instance, in EP 0230893 (Bracco).

Both Gd₂O₃ and meglumine are commercially available, for example from Sigma-Aldrich (PN 278513 and M9179 respectively), whereas the BOPTA ligand may be prepared, according to methods known to the ordinary practitioner, (*e.g*. WO 2007/031390 - Bracco Imaging SpA) and, if desired, stored in a solid form.

In this sense, a proper solid form of the BOPTA may be obtained according to procedures known in the art, such as, among others, by multiple crystallizations from acetone/water, next to the elution on a chromatographic resin, as disclosed in WO 2007/031390. In particular, said procedure contemplates first the isolation of the wet solid BOPTA, and then the final drying step under controlled temperature to obtain a final solid product with a satisfactory solvent content.

Alternatively, we have now found that the isolation of BOPTA in a convenient dried solid form may be achieved by dissolution of the aforementioned wet solid BOPTA in an aqueous medium, so to obtain an aqueous liquid composition, and submitting the latter to a spray-dry procedure. By that, the dried solid BOPTA may be readily collected in a short frame of time, avoiding the drying step, as formerly reported.

It is therefore an additional aspect of the present invention, a process for the preparation of a solid form of BOPTA, characterized by spray-drying a liquid composition of said compound.

Unless otherwise provided, said process may be conveniently performed substantially using the same devices and conditions as previously described for the gadobenate dimeglumine spray-drying.

By analogy, the term "liquid composition" in this further aspect of the invention has basically the same meaning as above, i.e. a solution, or even a suspension, of the selected compound (in this case BOPTA) in any appropriate solvent system.

As mentioned, a suitable BOPTA liquid composition for the spray-drying step may be obtained by dissolution of the wet BOPTA obtained according to W02007/031390 in a proper solvent system. Also in this respect, appropriate solvent systems are substantially the same as previously exemplified for the gadobenate dimeglumine spray-dry process. Thus, the isolation of the solid BOPTA may be carried out by spray-drying a corresponding liquid solution with a concentration preferably ranging from 7% w/w to 14% w/w (where % w/w means the mass percentage of the compound with respect to the mass of the total composition), at an inlet temperature of the device between about 120°C and 160°C, and an outlet temperature between about 60°C and 95°C.

The feed rate of the liquid composition might be promptly selected between 1200 and 2400 g/h at a feed temperature ranging from approximately 40°C to 50°C, by using, for instance, a MOBILE MINOR^{™} pilot plant.

The solid BOPTA thus collected may be conveniently used, for example, in the preparation of paramagnetic complexes, e.g. the previous mentioned gadobenate dimeglumine, by procedures known in the art and formerly reported.

As set forth in the experimental part herein below, all of the obtained data clearly support the consistency and reliability of the process of the present invention, being intended for the preparation of a solid form of gadobenate dimeglumine by spray-drying a corresponding aqueous solution. Moreover, the process substantially prevents the degradation of the powdered compound during the isolation and the eventual storage. From all the above, the skilled in the art will recognize that any modifications of the previously indicated reaction variables and conditions, are to be intended as a process optimization and, therefore, included in the scope of the present invention.

The invention will be now illustrated in more details by the Examples reported in the following Experimental Part, which are not to be intended as limitative to the scope of the invention in any manner.

### EXPERIMENTAL PART

### EXAMPLE 1: Solid form of gadobenate dimeglumine obtained by spray-drying

Spray-drying devices employed:
LAB PLANT SD04 spray dryer with co-current flow, equipped with a two fluid nozzle.
MOBILE MINOR^{™} pilot plant, with co-current flow and equipped with two fluid nozzle or pressure nozzle.

### Gadobenate dimeglumine general spray-drying procedure

The solution was fed to the spray-drying device at room temperature through a two fluid nozzle or pressure nozzle, which is located at the top of the chamber, and atomized into the chamber.

At the same time a hot air flow was blown into the chamber to dry the atomised particles.

The solid particles thus generated are subsequently separated from the exhausted air flow by collection through a cyclone.

The solid is gathered in a sample collector located at the bottom of the chamber.

### Example 1.1

842 g of a 0.485 M aqueous solution of gadobenate dimeglumine was fed to Lab Plant SD04 using the following parameters:
Tinlet 160 °C
Toutlet 87 °C
Feed rate: 8 g/min
310 g of gadobenate dimeglumine as white powder was obtained (yield 85%; KF 2.23%).

### Example 1.2

804 g of a 0.530 M aqueous solution of gadobenate dimeglumine was fed to Lab Plant SD04 using the following parameters:
Tinlet 160 °C
Toutlet 88 °C
Feed rate 8.8 g/min
333 g of gadobenate dimeglumine as white powder was obtained (yield 91%; KF 1.74%).

### Example 1.3

790 g of a 0.348 M aqueous solution of gadobenate dimeglumine was fed to Lab Plant SD04 using the following parameters:
Tinlet 160 °C
Toutlet 88 °C
Feed rate 5.9 g/min
208 g of gadobenate dimeglumine as white powder was obtained (yield 81.5%; KF 1.36%).

### Example 1.4

608 g of a 0.535 M aqueous solution of gadobenate dimeglumine was fed to Lab Plant SD04 using the following parameters:
Tinlet 195 °C
Toutlet 95 °C
Feed rate 6.1 g/min
232 g of gadobenate dimeglumine as white powder was obtained (yield 83.4%; KF 2.95%).

### Example 1.5

1300 g of a 0.5 M aqueous solution of gadobenate dimeglumine was fed to MOBILE MINOR^{™} pilot plant using the following parameters:
Tinlet 190 °C
Toutlet 100 °C
Feed rate 3.9 kg/h
406.1 g of gadobenate dimeglumine as white powder was obtained (yield 97%; KF 2.6%).

### Example 1.6

1500 g of a 0.5 M aqueous solution of gadobenate dimeglumine was fed to MOBILE MINOR^{™} pilot plant using the following parameters:
Tinlet 150 °C
Toutlet 80 °C
Feed rate 3.0 kg/h
608.3 g of gadobenate dimeglumine as white powder was obtained (yield 94%; KF 2.2%).

### EXAMPLE 2: comparative examples

Solid form of gadobenate dimeglumine obtained by alternative procedures

### Comparative Example 2.1: isolation by water evaporation

In a 1L reactor BOPTA (73.5 g; 143 mmol), N-methyl-glucamine (53.0 g; 271 mmol) and water (700 mL) were stirred at 50°C until complete dissolution. Gd₂O₃ (26.15 g; 72.1 mmol) was added and the suspension was stirred at 80°C for 75 min. At the end the mixture was filtered, the pH was adjusted to 7 and the solution was evaporated under vacuum to give a sticky glue-like residue.

The residue was dried at 25°C under vacuum (1 mmHg) with P₂O₅ obtaining a glassy hard solid difficult to collect due to its hardness.

### Comparative Example 2.2: isolation by lyophilization

100 mL of a 0.5 M aqueous solution of gadobenate dimeglumine (prepared according to the procedure described in the Comparative Example 1) were lyophilized using a Christ Alpha 1-4 lyophilizer at for 24h to give a glassy solid, whose recovering is troublesome and unprofitable, especially when applied to industrial scale amounts.

### Comparative Example 2.3: isolation by precipitation

5 mL of a 0.5 M gadobenate dimeglumine solution (prepared according to the procedure described in the Comparative Example 1) were added dropwise to 100 mL of 2-propanol, under stirring at room temperature.

A sticky gummy solid was obtained by precipitation and its recovering was problematic and annoying due to the unfavorable consistency of the solid form.

### Comparative Example 4: isolation by precipitation

4 mL of a 0.5 M gadobenate dimeglumine solution (prepared according to the procedure described in the Comparative Example 1) were added dropwise to 100 mL of ethanol kept under stirring at room temperature.

A white gummy solid was obtained by precipitation, but also in this case, its recovering was problematic and annoying due to the unfavorable consistency of the solid form.

### EXAMPLE 3: solid form of BOPTA obtained by spray-drying

Spray-drying device:
MOBILE MINOR^{™} pilot plant with co-current flow and equipped with two fluid nozzle or pressure nozzle

### BOPTA general spray-drying procedure

The same general procedure as the above gadobenate dimeglumine spray-drying procedure has been followed. An aqueous suspension or solution of BOPTA was fed to the spray-dryer at a temperature between about 45-50°C, with a co-current flow of air and equipped with two fluid nozzle.

The aqueous suspension or solution of BOPTA was obtained by solving an appropriate amount of wet solid BOPTA in an aqueous medium. The wet solid BOPTA is obtained by working in analogy to what disclosed in the previously mentioned W02007/031390: As a general reference, 452 g of an aqueous solution of the carboxylate sodium salt of N-[2-[(2-aminoethyl)amino]ethyl]-O-(phenylmethyl)serine (0.43 mol) were charged in a vessel of 3L with 92mL of water. The solution was heated to 55°C and reacted with 356 g of an 80% bromoacetic acid aqueous solution. The pH was kept at 11-12 by 30% (w/w) sodium hydroxide solution. The reaction was completed in about 5 h at 55°C and pH 11-12. The solution was cooled to 25°C and pH was adjusted to 5.5 with 34% HCl so to lead to an aqueous solution of the titled compound which was eluted on a chromatographic resin, concentrated, acidified and subsequently crystallized.After filtration, the wet solid obtained was dissolved in a proper amount of water and then subjected to a spray-drying procedure to get a final dried solid BOPTA.

### Example 3.1

920 g of a 14.0 % (w/w) BOPTA solution was fed to MOBILE MINOR^{™} pilot plant at 50°C using the following parameters:
Tinlet 160 °C
Toutlet 73 °C
Feed rate 2400 g/h
121.5 g of BOPTA as white powder was obtained (yield 94 %; KF 1.5 %).

### Example 2.2

930 g of a BOPTA suspension (14 % w/w) was fed to MOBILE MINOR^{™} pilot plant at 45°C using the following parameters:
Tinlet 140 °C
Toutlet 72 °C
Feed rate 1750 g/h
93.8 g of BOPTA as white powder was obtained (yield 72 %; KF 1.9 %).

## Claims

1. Process for the preparation of a solid form of gadobenate dimeglumine compound, comprising spray-drying a liquid composition of said compound.

2. Process according to claim 1 wherein the liquid composition is a solution or a suspension.

3. Process according to any one of the claims 1-2 wherein said liquid composition is an aqueous liquid composition.

4. Process according to claim 1-3 wherein the concentration of the liquid composition is between 0.2 M and 0.6 M.

5. Process according to claim 4 wherein said concentration is 0.5 M.

6. Process according to anyone of the preceding claims wherein the process is carried out at an inlet temperature comprised between 140°C and 280°C and at an outlet temperature between 70°C and 120°C.

7. Gadobenate dimeglumine in a solid form.

8. Compound according to claim 7 in an amorphous solid form.

9. Use of the compound of claim 7 for the preparation of a pharmaceutical formulation.

10. Pharmaceutical kit comprising gadobenate dimeglumine according to claim 7 and a liquid solution.

11. A pharmaceutical kit according to claim 10 wherein said liquid solution is water for injection.

12. Use of the pharmaceutical kit according to claims 10 and 11 for the preparation of an injectable pharmaceutical formulation.

13. Process for the preparation of a solid form of 4-carboxy-5,8,11-tris(carboxymethyl 1)-1-phenyl-2-oxa-5,8,11-triazatridecan-13-oic acid, **characterised by** spray-drying a liquid composition of said compound.

14. Process according to claim 12 wherein said liquid composition is an aqueous solution or suspension.

15. Process according to claim 13 wherein the concentration of said solution is from 7% w/w to 14%w/w.
